# EUROPEAN PATENT SPECIFICATION

(11) **EP 3 351 920 B1**
(45) Date of publication and mention of the grant of the patent: **01.11.2023**
(21) Application number: 16845916.2
(22) Date of filing: 06.09.2016
(51) Int. Cl.: G01N 1/22, G01N 1/28, B01D 53/04, B01J 20/06, B01J 20/28, B01J 20/32, B82Y 30/00, B01J 20/20, G01N 33/00, G01N 1/40

(54) **CHEMICAL SUBSTANCE CONCENTRATOR AND CHEMICAL SUBSTANCE DETECTION DEVICE**
KONZENTRATOR EINER CHEMISCHEN SUBSTANZ UND VORRICHTUNG ZUM NACHWEIS EINER CHEMISCHEN SUBSTANZ
CONCENTRATEUR DE SUBSTANCE CHIMIQUE ET DISPOSITIF DE DÉTECTION DE SUBSTANCE CHIMIQUE

(30) Priority: 18.09.2015 JP 2015184559
(43) Date of publication of application: 25.07.2018
(73) Proprietor: Panasonic Holdings Corporation, Osaka 571-8501 (JP)
(72) Inventor: NAKAO, Atsuo, Osaka-shi Osaka 540-6207 (JP); HANAI, Yosuke, Osaka-shi Osaka 540-6207 (JP)
(74) Representative: Grünecker Patent- und Rechtsanwälte PartG mbB
(86) International application number: PCT/JP2016/004053
(87) International publication number: WO 2017/047041

(56) References cited:
- WO-A1-2009/104202
- WO-A1-2016/103561
- JP-A- H10 227 725
- JP-A- H11 287 743
- JP-A- S61 286 742
- JP-A- 2004 148 211
- JP-A- 2006 187 857
- JP-A- 2010 197 387
- JP-A- 2014 504 740
- US-A1- 2004 100 269
- US-A1- 2005 245 836
- US-A1- 2008 093 226
- US-A1- 2012 119 760
- US-A1- 2015 010 442
- KEAT GHEE ONG ET AL: "A Wireless, Passive Carbon Nanotube-Based Gas Sensor", IEEE SENSORS JOURNAL, IEEE SERVICE CENTER, NEW YORK, NY, US, vol. 2, no. 2, 1 April 2002 (2002-04-01), XP011065645, ISSN: 1530-437X

## Description

### TECHNICAL FIELD

The present disclosure relates to techniques of analyzing and detecting a chemical substance contained in a gas.

### BACKGROUND ART

Techniques of analyzing a chemical substance contained in a gas are disclosed in, for example, PTL 1 and PTL 2. PTL 1 discloses an apparatus for analyzing an organic substance contained in a gas in an electric power apparatus. In this analyzing apparatus, a gas passes through a pipe while the temperature of a trap is constant, so that an organic substance in the gas is adsorbed onto an adsorbent. Then, the trap is heated so that the adsorbed organic substance is introduced into a detector. PTL 2 discloses a detection device for a trace amount of an analyte employing an adsorbent substance capable of adsorbing the analyte and desorbing the concentrated analyte.

US 2005/245836 A1 relates to a nanoelectronic capnometer adapter.

Keat Ghee Ong et al is about "A Wireless, Passive Carbon Nanotube-Based Gas Sensor" (IEEE sensors journal, vol. 2, no. 2, 1 April 2002).

WO 2009/104202 A1 relates to a device and a method to use single walled carbon nanotube composites for gas sensing applications.

US 2004/100269 A1 relates to a nanotube sensor.

US 2008/093226 A1 relates to an ammonia nanosensors, and an environmental control system.

US 2015/010442 relates to a device comprising an adsorbent which is configured to self-heat due to Joule's heat when a current passes through the adsorbent.

US 2012/119760 relates to a device comprising an adsorber made out of nanowires.

### CITATION LIST

### PATENT LITERATURE

PTL 1: Japanese Patent Laid-Open Publication No. 2001-296218
PTL 2: Japanese Patent Laid-Open Publication No. 2002-518668

### SUMMARY

A chemical substance concentrator according to the present invention is defined in claim 1.

A chemical substance detection device according to the present invention is defined in claim 11.

The chemical substance concentrator and the chemical substance detection device according to the present disclosure can efficiently desorb the adsorbed chemical substance. Further advantageous embodiments are defined in dependent claims.

### BRIEF DESCRIPTION OF DRAWINGS

FIG. 1 is a schematic perspective view of a chemical substance concentrator according to an exemplary embodiment.
FIG. 2 is a schematic cross-sectional view of the chemical substance concentrator according to the embodiment.
FIG. 3 is a schematic cross-sectional view another chemical substance concentrator not being part of the present invention.
FIG. 4 is a schematic cross-sectional view of another chemical substance concentrator according to the embodiment.
FIG. 5 is a top view of adsorbents according to the embodiment for illustrating an arrangement of the absorbents.
FIG. 6 is a top view of the adsorbents according to the embodiment for illustrating another arrangement example of the absorbents.
FIG. 7 is a top view of the adsorbents according to the embodiment for illustrating still another arrangement of the absorbents.
FIG. 8 is a top perspective view of a chemical substance detection device according to the embodiment.

### DETAIL DESCRIPTION OF EXEMPLARY EMBODIMENT

In accordance with the above-mentioned conventional configurations, it is necessary to use an external heating means such as an external heater to introduce an adsorbed chemical substance into a detector. If a chemical substance is desorbed without using such a heating means, the desorption is insufficient.

However, when an adsorbent is heated using an external heater, part of heat generated by the external heater diffuses into the surroundings, and accordingly the heat is not conducted to the adsorbent. In other words, the conventional configurations using an external heater cause a larger heat loss of the external heater, and therefore, the adsorbent cannot be efficiently heated. The heating efficiency of an adsorbent affects the ease of desorption of an adsorbed substance.

As described above, the conventional configurations have a problem that an adsorbed chemical substance cannot be efficiently desorbed.

A chemical substance concentrator and a chemical substance detection device according to an exemplary embodiment of the present disclosure will be detailed below with reference to drawings. Any of embodiments described below shows a preferable specific example of the present disclosure. Therefore, the numerical values, shapes, materials, constituent elements, the arrangement and connection of the constituent elements, and the like shown in the following embodiments are mere examples, and do not limit the scope of the present disclosure. Therefore, among the constituent elements in the following embodiments, constituent elements not recited in any one of independent claims each indicating the broadest concept of the present invention are described as arbitrary constituent elements.

In addition, the drawings are schematic drawings, and are not necessarily exact depictions. In the drawings, substantially the same constituent elements are assigned the same reference numerals, and duplicate descriptions regarding these elements are omitted or simplified.

### Exemplary Embodiments

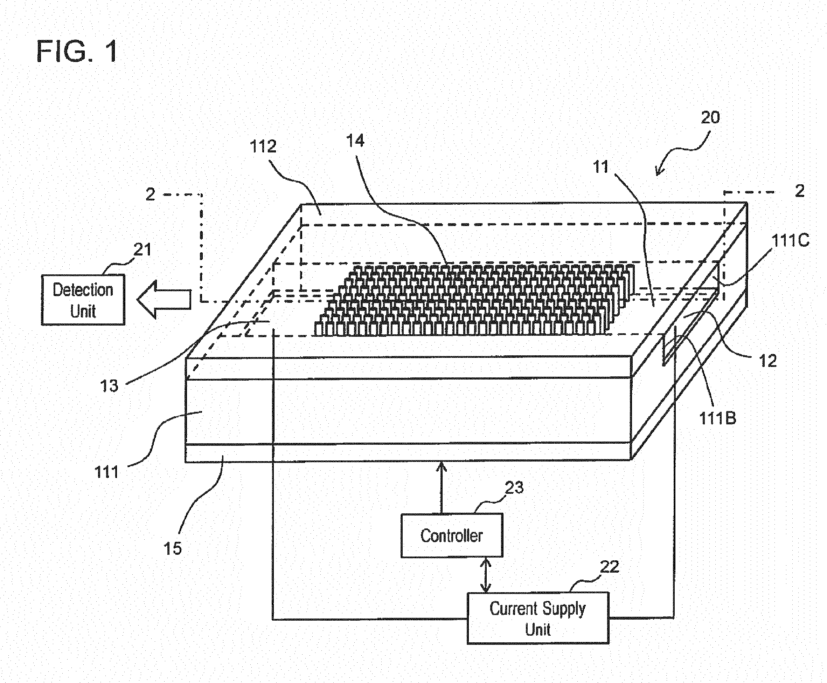

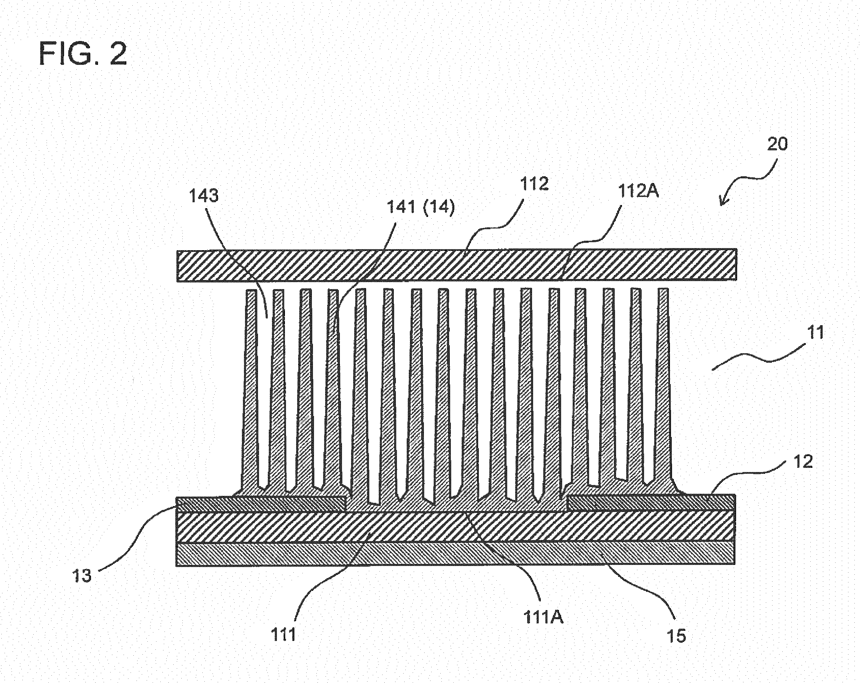

A chemical substance concentrator and a chemical substance detection device according to one aspect of the present disclosure will be described with reference to FIG. 1 and FIG. 2.

FIG. 1 is a top perspective view of chemical substance concentrator 20. FIG. 2 is a schematic cross-sectional view of chemical substance concentrator 20 on line 2-2 shown in FIG. 1.

Chemical substance concentrator 20 is configured to concentrate a chemical substance contained in a gaseous sample flowing into the concentrator. The chemical substance concentrated by chemical substance concentrator 20 is detected by, for example, detection unit 21 disposed downstream from chemical substance concentrator 20.

The gaseous sample may be an exhaled breath of human and animals and exhaust gases from cars and factories. The chemical substance may be volatile organic compounds, such as ketones, amines, alcohols, aromatic hydrocarbons, aldehydes, esters, organic acids, hydrogen sulfide, methyl mercaptan, or disulfide.

Chemical substance concentrator 20 includes flow passage 11 that allows the gaseous sample containing the chemical substance to flow through the passage, first electrode 12 disposed on first inner wall 111A of flow passage 11, second electrode 13 being disposed on first inner wall 111A and apart from first electrode 12, adsorbent 14 disposed on first inner wall 111A so as to contact the first electrode and the second electrode, and cooling unit 15 that cools adsorbent 14.

Flow passage 11 includes, e.g. upper substrate 112 and lower substrate 111 having a groove therein. A bottom surface of the groove provided in lower substrate 111 constitutes first inner wall 111A facing flow passage 11. One side surface of the groove constitutes second inner wall 111B. Another side surface of the groove constitutes third inner wall 111C. A lower surface of upper substrate 112 which covers a top of the groove constitutes fourth inner wall 112A. Flow passage 11 has a quadrangular prism shape surrounded by first inner wall 111A, second inner wall 111B, third inner wall 111C, and fourth inner wall 112A which extend in an extending direction in which flow passage 11 extends. Flow passage 11 may have a polygonal prism shape surrounded by another inner wall in addition to the above-mentioned four inner walls. Each of lower substrate 111 and upper substrate 112 are made of, for example, resin or metal.

First electrode 12 is disposed on first inner wall 111A of flow passage 11. First electrode 12 is made of, for example, gold, copper, platinum, or carbon.

Second electrode 13 is disposed on first inner wall 111A of flow passage 11. Second electrode 13 is made of, for example, gold, copper, platinum, or carbon. First electrode 12 and second electrode 13 may be made of the same material.

First electrode 12 and second electrode 13 are disposed on the same surface of flow passage 11. Second electrode 13 is apart from first electrode 12 so as not to directly contact first electrode 12. Furthermore, first electrode 12 and second electrode 13 are arranged in a flow direction of the gaseous sample in which the gaseous sample flows.

First electrode 12 and second electrode 13 may be arranged in a direction perpendicular to the flow direction of the gaseous sample.

Adsorbent 14 adsorbs a chemical substance contained in the gaseous sample.

Adsorbent 14 is disposed on first inner wall 111A of flow passage 11. Adsorbent 14 contacts first electrode 12 and second electrode 13. Adsorbent 14 continuously contacts first inner wall 111A between first electrode 12 and second electrode 13.

Adsorbent 14 is made of an aggregate of conductive nanowires 141. In other words, adsorbent 14 includes a group of conductive nanowires 141. Nanowires 141 are made of, for example, conductive metal oxide. Adsorbent 14 has voids 143 therein each provided between the nanowires. The chemical substance contained in the gaseous sample is adsorbed onto nanowires 141 while passing through voids 143.

Conductive nanowires 141 may be made of metal oxides, such as SnOz, ZnO, In₂O₃, In₂₋ₓSnₓO₃ (for example, 0.1≤x≤0.2), NiO, CuO, TiO₂, or SiOz, metals, such as Al, Ag, Au, Pd, or Pt, carbon, or silicon. Nanowires made of carbon may be made of, e.g. carbon nanotubes. In other words, adsorbent 14 is made of a material having conductivity and a resistance enough to heat adsorbent 14 itself due to a Joule effect effectively.

Alternatively, nanowires 141 may be made of resin, for example, having surfaces coated with conductive metal oxide. The conductive metal oxide coating the nanowires allows adsorbent 14 to have conductivity.

Adjacent nanowires 141 of adsorbent 14 are joined together at bases of adjacent nanowires 141 on the first inner wall 111A. Adjacent nanowires 141 may be joined together at their ends or middle portions, or may be joined together at a combination of their bases, their ends, and their middle portions.

Nanowires 141 thus joined together provide adsorbent 14 with electrical conductivity as a whole. First electrode 12 and second electrode 13 are electrically connected to each other via adsorbent 14.

When a current passes through adsorbent 14 via first electrode 12 and second electrode 13, adsorbent 14 generates Joule's heat. Adsorbent 14 causes self-heating due to Joule's heat. A chemical substance adsorbed onto adsorbent 14 is desorbed from adsorbent 14 due to the heat generated by adsorbent 14. Conductive adsorbent 14 functions as a heater as well as adsorbing the chemical substance.

When adsorbent 14 functions as a heater, chemical substance concentrator 20 allows the adsorbed chemical substance to be desorbed without using an external heater which consumes a lot of power.

The heat generated by adsorbent 14 is directly used for desorbing the chemical substance adsorbed onto adsorbent 14. In other words, chemical substance concentrator 20 has higher thermal efficiency than a device that employs an external heater.

Heat generated by an external heater often diffuses into ambient area, accordingly not efficiently transmitting to adsorbent 14. Such a poor thermal efficiency requires extra electric power for heating adsorbent 14, thus causing a problem that the device consumes more power.

Chemical substance concentrator 20 according to the embodiment utilizes the heat generated by adsorbent 14 to desorb the chemical substance, hence reducing heat loss in heating of adsorbent 14. Chemical substance concentrator 20 can thus concentrate the chemical substance efficiently.

An external heater may consume several tens to several hundreds of mW of electric power. In Micro Electro Mechanical Systems (MEMS) technique, for example, a Pt-wire resistance heating heater consumes several mW or more power. In contrast, chemical substance concentrator 20 according to the present embodiment can desorb the chemical substance with, e.g. power equal to or smaller than 10 µW.

Thus, chemical substance concentrator 20 can concentrate the chemical substance with lower power consumption. Since not requiring an external heater, chemical substance concentrator 20 can have a small size.

First electrode 12 and second electrode 13 are connected to current supply unit 22 that supplies a current to adsorbent 14. Controller 23 that controls a current flowing through adsorbent 14 is connected to current supply unit 22.

Cooling unit 15 is configured to cool adsorbent 14. Upon being cooled, adsorbent 14 can adsorb a chemical substance more efficiently.

Cooling unit 15 is disposed on a surface of lower substrate 111 opposite to the surface lower substrate 111 constituting first inner wall 111A. Cooling unit 15 may be implemented by a Peltier element. In this case, controller 23 connected to cooling unit 15 controls the cooling of adsorbent 14.

As long as cooling unit 15 can cool adsorbent 14, cooling unit 15 may be disposed at any position. For example, cooling unit 15 may be disposed inside flow passage 11. Alternatively, cooling unit 15 may be disposed on first electrode 12 or second electrode 13. Each of the electrodes is made of metal having high heat conductivity, and facilitating the cooling of adsorbent 14 efficiently. In the case where cooling unit 15 is disposed on first electrode 12, an insulating layer may be provided between cooling unit 15 and first electrode 12. Similarly, in the case where cooling unit 15 is disposed on second electrode 13, an insulating layer may be provided between cooling unit 15 and second electrode 13.

In the case where the chemical substance is adsorbed sufficiently onto adsorbent 14, the apparatus does not necessarily include cooling unit 15.

Adsorbent 14 in accordance with the present embodiment is made of nanowires 141 since adsorbent 14 has a larger specific surface area, and accordingly, yields higher concentration (adsorption) efficiency. Nanowires 141 have small heat capacity, and accordingly have a temperature changes drastically with low power consumption.

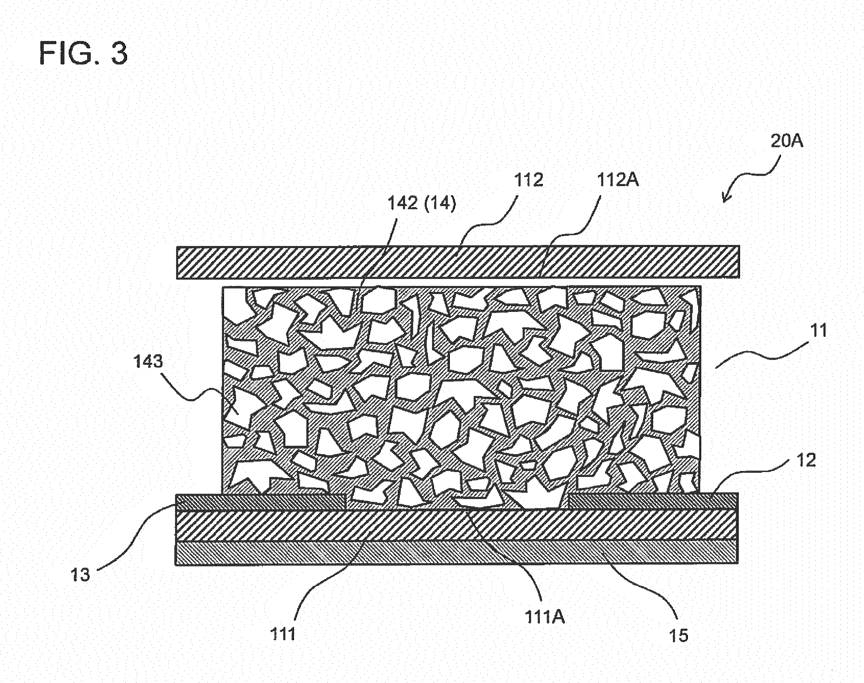

In an example not being part of the present invention, the adsorbent 14 is not necessarily made of nanowires 141. As illustrated in chemical substance concentrator 20A shown in FIG. 3, adsorbent 14 may be made of porous body 142 having voids 143 allowing the gaseous sample to pass through voids 143. Porous body 142 is made of material, such as conductive metal oxide, identical to that of nanowire 141. Porous body 142 has a structure in which a lot of voids 143 are randomly provided therein. Thus, a lot of conductive paths between first electrode 12 and second electrode 13 are provided besides a conduction path formed at a portion of porous body 142 contacting first inner wall 111A. Porous body 142 having a lot of conductive paths reduces a temperature variation between portions of
Porous body 142 may be formed by coating the surface of a porous structure made of carbon or resin with conductive metal oxide, for example. This configuration provides a structure for heating the surface of porous body 142 onto which the chemical substance is adsorbed, thus allowing the chemical substance to be efficiently desorbed. Porous body 142 coated with the conductive material has a small volume of the conductive material of porous body 142. This configuration allows porous body 142 to reduce power consumed by self-heating due to the Joule effect.

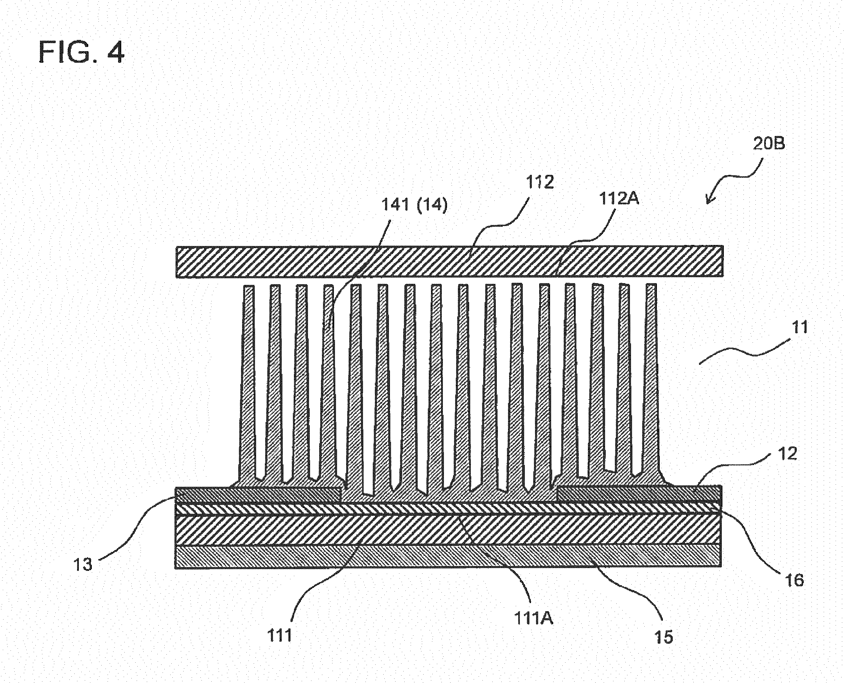

FIG. 4 is a cross-sectional view of another example of chemical substance concentrator 20.

Chemical substance concentrator 20B includes thermal insulating layer 16 provided on first inner wall 111A of flow passage 11. Thermal insulating layer 16 contacts adsorbent 14. Thermal insulating layer 16 prevents heat generated by adsorbent 14 from transmitting to the outside via lower substrate 111. Thermal insulating layer 16 may be made of resin material, such as epoxy resin, polyimide, polyethylene terephthalate, polystyrene, or polycarbonate. Alternatively, thermal insulating layer 16 may be made of metal oxide material, such as ZrO₂ or Al₂TiO₅, glass material; or porous material, such as silica aerogel or expandable polymer.

A gap is provided between adsorbent 14 and upper substrate 112. The gap between adsorbent 14 and upper substrate 112 prevents heat generated by adsorbent 14 from transmitting to upper substrate 112.

Adsorbent 14 may contact upper substrate 112. In this case, a thermal insulating layer may be provided further on a surface of upper substrate 112. The thermal insulating layer contacts adsorbent 14. This configuration prevents heat generated by adsorbent 14 from transmitting to upper substrate 112 even when adsorbent 14 contacts upper substrate 112.

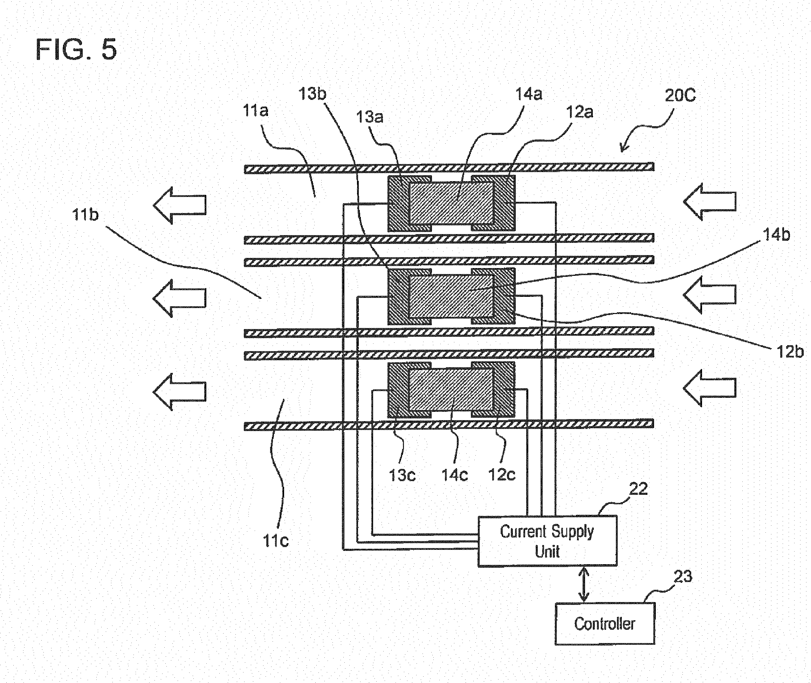

FIG. 5 is a schematic diagram of adsorbents 14 for illustrating an arrangement of adsorbents 14.

Chemical substance concentrator 20C includes plural flow passages 11a, 11b, and 11c. Adsorbents 14a, 14b, and 14c are disposed inside the flow passages 11a, 11b, and 11c, respectively. Adsorbent 14a is connected to first electrode 12a and second electrode 13a. Adsorbent 14b is connected to first electrode 12b and second electrode 13b. Adsorbent 14c is connected to first electrode 12c and second electrode 13c.

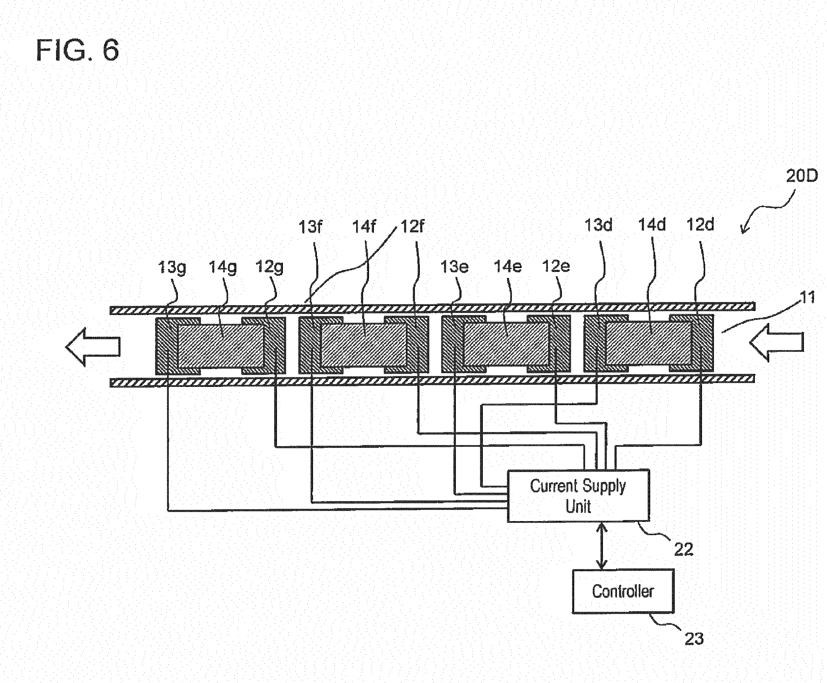

FIG. 6 is a schematic diagram of adsorbents 14 for illustrating another arrangement of adsorbents 14.

Chemical substance concentrator 20D includes plural adsorbents 14d, 14e, 14f, and 14g. Adsorbents 14d to 14g are connected to first electrodes 12d to 12g and second electrodes 13d to 13g, respectively.

In other words, adsorbents 14d to 14g are disposed separately from each other in one flow passage 11. Adsorbents 14d to 14g are arranged in a flow direction in which the gaseous sample flows.

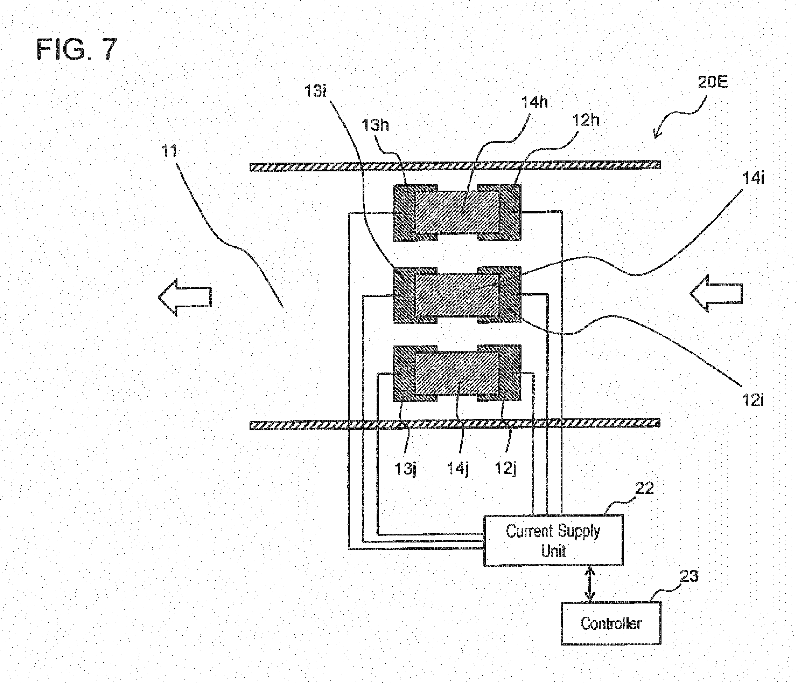

FIG. 7 is a schematic diagram of adsorbents 14 for illustrating still another arrangement of adsorbents 14.

Chemical substance concentrator 20E includes plural adsorbents 14b, 14i, and 14j. Adsorbents 14h to 14j are connected to first electrodes 12h to 12j and second electrodes 13h to 13j, respectively.

In other words, adsorbents 14h to 14j are disposed separately from each other in single flow passage 11. Adsorbents 14h to 14j are arranged in a direction perpendicular to the flow direction of the gaseous sample.

Each of first electrodes 12a to 12j and second electrodes 13a to 13j, which are connected to adsorbents 14a to 14j is electrically connected to current supply unit 22. Current supply unit 22 supplies a current selectively to first electrodes 12a to 12j and second electrodes 13a to 13j connected to adsorbents 14a to 14j, respectively.

In the case where plural adsorbents 14d to 14g are disposed separately from each other in single flow passage 11, as illustrated in FIG. 6, adsorbents 14d to 14g may be made of materials different from each other, or surfaces of the adsorbents 14d to 14g may be modified differently from each other. This configuration provides different adsorbents 14d to 14g to adsorb different chemical substances. Thus, chemical substance concentrator 20D can selectively adsorb and concentrate the chemical substances contained in the gaseous sample. In the case where plural adsorbents 14h to 14j are disposed separately from each other in single flow passage 11, as illustrated in FIG. 7, adsorbents 14h to 14j may be made of different materials from each other, or surfaces of adsorbents 14h to 14j may be modified differently from each other. This configuration allows adsorbents 14h to 14j to adsorb chemical substances. Thus, chemical substance concentrator 20E can selectively adsorb and concentrate chemical substances contained in the gaseous sample.

For example, chemical substances are easily adsorbed onto substances having the same polarity. A chemical substance made of highly polar molecules is easily adsorbed onto adsorbent 14 having a highly polar surface. In contrast, a chemical substance made of non-polar molecules is easily adsorbed onto adsorbent 14 having a non-polar surface. Thus, a strength with which a chemical substance is adsorbed depends on the material of adsorbent 14.

Variety to the property of adsorbent 14 due to, for example, the material or surface modification thereof allows chemical substance concentrator 20C to cause chemical substances to be adsorbed selectively onto adsorbents 14a to 14c. Chemical substance concentrator 20D allows chemical substances to be adsorbed selectively onto adsorbents 14d to 14g. Chemical substance concentrator 20E allows chemical substances to be absorbed selectively onto adsorbents 14h to 14j.

In chemical substance concentrator 20C including adsorbents 14a to 14c disposed separately from each other, current supply unit 22 for supplying a current to adsorbents 14a to 14c may supply a current selectively to first electrodes 12a to 12c and second electrodes 13a to 13c disposed in adsorbents 14a to 14c, respectively. Similarly, in chemical substance concentrator 20D, current supply unit 22 for supplying a current to adsorbents 14d to 14g may supply a current selectively to first electrodes 12d to 12g and second electrodes 13d to 13g disposed in adsorbents 14d to 14g, respectively. Similarly, in chemical substance concentrator 20E, current supply unit 22 for supplying a current to adsorbents 14h to 14j may supply a current selectively to first electrodes 12h to 12j and second electrodes 13h to 13j disposed in adsorbents 14h to 14j, respectively.

This configuration allows the timing of desorbing chemical substances adsorbed onto respective adsorbents 14a to 14c, 14d to 14g, and 14h to 14j to be controlled on respective adsorbents. Thus, chemical substance concentrators 20C to 20E can cause only a chemical substance as a detection target to be desorbed from adsorbents 14a to 14c, 14d to 14g, or 14h to 14j, and sent to detection unit 21.

When adsorbent 14 adsorbs a chemical substance, the electric resistance of adsorbent 14 changes. Hence, by detecting this change in the electric resistance, a chemical substance can be identified. For identifying a chemical substance adsorbed onto adsorbent 14, it is not necessary to use a precise analyzer as detection unit 21 arranged downstream. This further reduces the size of the apparatus.

First electrodes 12a to 12c, 12d to 12g, and 12h to 12j and second electrodes 13a to 13c, 13d to 13g, and 13h to 13j, which are connected to adsorbents 14a to 14c, 14d to 14g, and 14h to 14j, respectively, may not be separate electrodes. For example, adsorbents 14a to 14c, 14d to 14g, and 14h to 14j may be disposed so as to be connected to single first electrode 12 and single second electrode 13.

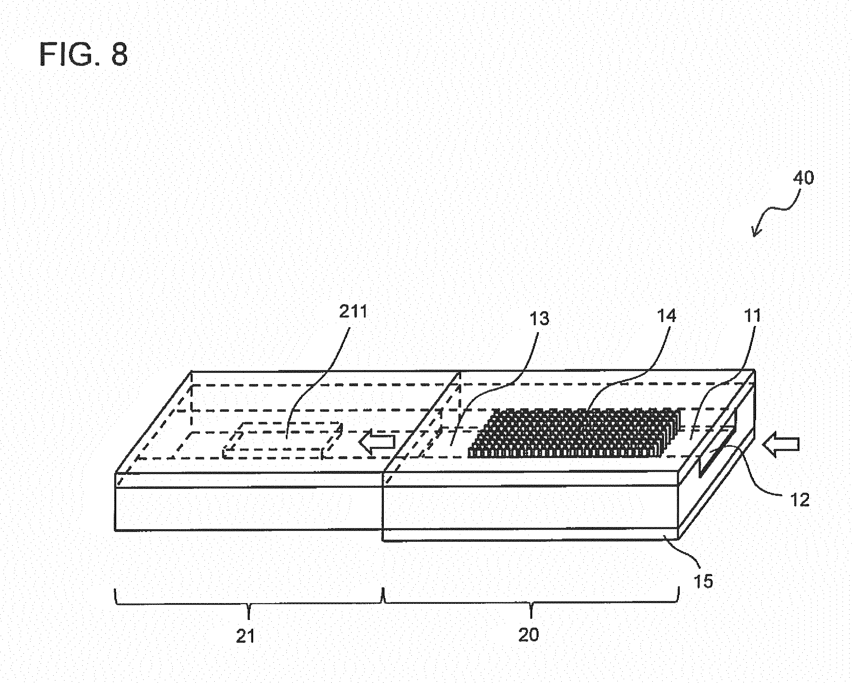

FIG. 8 is a top perspective view of chemical substance detection device 40 according to the embodiment. A gaseous sample flows in the direction of the arrow.

Chemical substance detection device 40 includes detection unit 21 which is connected subsequently to chemical substance concentrator 20, that is, located downstream from chemical substance concentrator 20. Detection unit 21 includes detection element 211 in flow passage 11.

Detection element 211 may be implemented by a semiconductor sensor, an electrochemical sensor, an optical sensor, or a biosensor using a surface acoustic wave element or a field-effect transistor.

Chemical substance detection device 40 is configured to have a chemical substance contained in a gaseous sample concentrated by chemical substance concentrator 20, and to detect the chemical substance concentrated by detection unit 21. Thus, chemical substance detection device 40 can detect the chemical substance at sufficient sensitivity.

Chemical substance concentrator 20 according to the embodiment thus detects the chemical substance.

In chemical substance concentrator 20 according to the embodiment, current supply unit 22 supplies a current flowing through adsorbent 14. This configuration can monitor the electric resistance of adsorbent 14. The electric resistance of adsorbent 14 changes when adsorbent 14 adsorbs a chemical substance. For example, in the case where adsorbent 14 is made of metal oxide, the amount of oxygen contained in the surface of adsorbent 14 changes in accordance with the amount of the adsorbed chemical substance. This configuration changes the electric resistance of adsorbent 14. Alternatively, also in the case where adsorbent 14 is made of material, such as silicon, other than metal oxide, if the adsorbed substance has a polarity, the electric resistance of adsorbent 14 changes in accordance with the amount of the adsorbed chemical substance. Thus, chemical substance concentrator 20 according to the embodiment can detect the chemical substance adsorbed onto adsorbent 14.

Chemical substance concentrator 20 may include an adsorption amount estimation unit for estimating the amount of the chemical substance adsorbed onto adsorbent 14 based on a change in the electric resistance of adsorbent 14. For example, current supply unit 22 illustrated in FIG. 1 may function as a measurement unit for measuring the value of a current flowing through adsorbent 14. Controller 23 may function as the adsorption amount estimation unit.

In this case, controller 23 stores a previously-learned relationship between the amount of the absorbed chemical substance and the change in the electric resistance of adsorbent 14. Then, the change in the electric resistance of adsorbent 14 is determined based on the value of the current flowing through adsorbent 14 which is measured by the measurement unit. Based on the change in the electric resistance of adsorbent 14, controller 23 estimates the amount of the chemical substance adsorbed onto adsorbent 14 with reference to the relationship stored relationship between the amount of the chemical substance adsorbed and the change in the electric resistance of adsorbent 14. The adsorption amount estimation unit allows, for example, the timing of desorbing the adsorbed chemical substance to be controlled appropriately.

The chemical substance concentrator and the chemical substance detection device according to one or more aspects according to the embodiment have been described, but the present disclosure is not limited to these embodiments. Various modifications to the embodiment that can be conceived by those skilled in the art and forms configured by combining constituent elements in different embodiments may be included within the scope of one or more of the aspects, unless such modifications and forms depart from the present invention as claimed.

### INDUSTRIAL APPLICABILITY

A chemical substance concentrator according to the present disclosure is useful for, for example, a small chemical sensor capable of detecting volatile organic compounds in environment.

### REFERENCE MARKS IN THE DRAWINGS

11 flow passage
111 lower substrate
111A first inner wall
112 upper substrate
12, 12a, 12b, 12c, 12d, 12e, 12f, 12g, 12h, 12i first electrode
13, 13a, 13b, 13c, 13d, 13e, 13f, 13g, 13h, 13i second electrode
14, 14a, 14b, 14c, 14d, 14e, 14f, 14g, 14h, 14i adsorbent
141 nanowire
142 porous body
143 void
15 cooling unit
16 thermal insulating layer
20, 20A, 20B, 20C, 20D, 20E chemical substance concentrator
21 detection unit
211 detection element
22 current supply unit
23 controller
40 chemical substance detection device

## Claims

1. A chemical substance concentrator comprising:
a flow passage (11) that allows a gaseous sample containing a chemical substance to flow through the flow passage;
a first electrode (12, 12a-12i) disposed on a first inner wall (111A) of the flow passage (11);
a second electrode (13, 13a-13i) disposed on the first inner wall (111A), the second electrode (13, 13a-13i) being apart from the first electrode (12, 12a-12i); and
a conductive adsorbent (14, 14a-14i) contacting the first electrode (12, 12a-12i), the second electrode (13, 13a-13i), and the first inner wall (111A) between the first electrode (12, 12a-12i) and the second electrode (13, 13a-13i),
wherein the adsorbent (14) is configured to adsorb the chemical substance,
the adsorbent (14) is made of an aggregate of conductive nanowires (141),
the adsorbent (14) includes voids (143) therein each provided between the nanowires,
adjacent nanowires (141) of the adsorbent (14) are joined together at bases of adjacent nanowires (141) on the first inner wall (111A) so as to provide the adsorbent (14) with electrical conductivity as a whole, the first electrode and the second electrode are electrically connected to each other via the adsorbent (14),
the adsorbent (14, 14a-14i) is configured to self-heat due to Joule's heat when a current passes through the adsorbent (14, 14a-14i) via the first electrode (12, 12a-12i) and the second electrode (13, 13a-13i), and
the adsorbent is configured to desorb the adsorbed chemical substance from the adsorbent (14) due to the heat generated by the adsorbent (14).

2. The chemical substance concentrator according to claim 1, wherein the nanowires (141) are made of metal oxide.

3. The chemical substance concentrator according to claim 1, wherein the nanowires (141) are made of carbon nanotubes coated with metal oxide.

4. The chemical substance concentrator according to claim 1, further comprising a cooling unit (15) for cooling the adsorbent (14, 14a-14i).

5. The chemical substance concentrator according to claim 1, wherein the first inner wall (111A) includes a thermal insulating layer (16) contacting the adsorbent (14, 14a-14i).

6. The chemical substance concentrator according to claim 1, wherein the flow passage (11) has a plurality of inner walls (111A-111C), and the first inner wall (111A) is one of the plurality of inner walls (111A-111C).

7. The chemical substance concentrator according to claim 1, wherein the adsorbent (14, 14a-14i) comprises a plurality of adsorbents disposed separately from each other in the flow passage (11).

8. The chemical substance concentrator according to claim 7, wherein the plurality of adsorbents are made of materials different from each other, or have surfaces modified differently from each other.

9. The chemical substance concentrator according to claim 7 or 8,
wherein each of the first electrode (12, 12a-12i) and the second electrode (13, 13a-13i) is at respective one of the plurality of adsorbents,
the chemical substance concentrator further comprising a current supply unit (22) that supplies a current selectively to each of the first electrode (12, 12a-12i) and the second electrode (13a, 13a-13i) disposed in the respective one of the plurality of adsorbents.

10. The chemical substance concentrator according to claim 1, further comprising:
a measurement unit that measures a value of a current flowing through the adsorbent (14, 14a-14i); and
an adsorption amount estimation unit that estimates an amount of the chemical substance adsorbed to the adsorbent (14, 14a-14i) by determining, based on the current value, a change in an electric resistance of the adsorbent (14, 14a-14i).

11. A chemical substance detection device comprising:
the chemical substance concentrator (20, 20A-20E) according to claim 1; and
a detection unit (21) that detects the chemical substance concentrated by the chemical substance concentrator (20, 20A-20E).

## Patentansprüche

1. Konzentrator für eine chemische Substanz, der umfasst:
einen Strömungskanal (11), der zulässt, dass eine gasförmige Probe, die eine chemische Substanz enthält, den Strömungskanal durchströmt;
eine erste Elektrode (12, 12a-12i), die an einer ersten Innenwand (111A) des Strömungskanals (11) angeordnet ist;
eine zweite Elektrode (13, 13a-13i), die an der ersten Innenwand (111A) angeordnet ist, wobei die zweite Elektrode (13, 13a-13i) von der ersten Elektrode (12, 12a-12i) getrennt ist; sowie
ein leitfähiges Adsorptionsmittel (14, 14a-14i), das mit der ersten Elektrode (12, 12a-12i), der zweiten Elektrode (13, 13a-13i) sowie der ersten Innenwand (111 A) zwischen der ersten Elektrode (12, 12a-12i) und der zweiten Elektrode (13, 13a-13i) in Kontakt ist,
wobei das Adsorptionsmittel (14) so ausgeführt ist, dass es die chemische Substanz adsorbiert,
das Adsorptionsmittel (14) aus einem Aggregat leitfähiger Nanodrähte (141) besteht,
das Adsorptionsmittel (14) Hohlräume (143) darin einschließt, die jeweils zwischen den Nanodrähten vorhanden sind,
benachbarte Nanodrähte (141) des Adsorptionsmittels (14) an unteren Enden benachbarter Nanodrähte (141) an der ersten Innenwand (111A) so miteinander verbunden sind, das dem Adsorptionsmittel (14) insgesamt elektrische Leitfähigkeit verliehen wird, wobei die erste Elektrode und die zweite Elektrode über das Adsorptionsmittel (14) elektrisch miteinander verbunden sind,
das Adsorptionsmittel (14, 14a-14i) so ausgeführt ist, dass es sich aufgrund von Joulescher Wärme selbst erwärmt, wenn ein Strom über die erste Elektrode (12, 12a-12i) und die zweite Elektrode (13, 13a-13i) durch das Adsorptionsmittel (14, 14a-14i) fließt, und
das Adsorptionsmittel so ausgeführt ist, dass es die adsorbierte chemische Substanz aufgrund der von dem Adsorptionsmittel (14) erzeugten Wärme aus dem Adsorptionsmittel (14) desorbiert.

2. Konzentrator für eine chemische Substanz nach Anspruch 1, wobei die Nanodrähte (141) aus Metalloxid bestehen.

3. Konzentrator für eine chemische Substanz nach Anspruch 1, wobei die Nanodrähte (141) aus mit Metalloxid beschichteten Kohlenstoffnanoröhrchen bestehen.

4. Konzentrator für eine chemische Substanz nach Anspruch 1, der des Weiteren eine Kühl-Einheit (15) zum Kühlen des Adsorptionsmittels (14, 14a-14i) umfasst.

5. Konzentrator für eine chemische Substanz nach Anspruch 1, wobei die erste Innenwand (111 A) eine wärmeisolierende Schicht (16) einschließt, die mit dem Adsorptionsmittel (14, 14a-14i) in Kontakt ist.

6. Konzentrator für eine chemische Substanz nach Anspruch 1, wobei der Strömungskanal (11) eine Vielzahl von Innenwänden (111A-111C) aufweist und die erste Innenwand (111 A) eine der Vielzahl von Innenwänden (111A-111C) ist.

7. Konzentrator für eine chemische Substanz nach Anspruch 1, wobei das Adsorptionsmittel (14, 14a-14i) eine Vielzahl von Adsorptionsmitteln umfasst, die getrennt voneinander in dem Strömungskanal (11) angeordnet sind.

8. Konzentrator für eine chemische Substanz nach Anspruch 7, wobei die Vielzahl von Adsorptionsmitteln aus voneinander verschiedenen Materialien bestehen oder Oberflächen aufweisen, die voneinander verschieden modifiziert sind.

9. Konzentrator für eine chemische Substanz nach Anspruch 7 oder 8,
wobei sich die erste Elektrode (12, 12a-12i) und die zweite Elektrode (13, 13a-13i) jeweils an einem entsprechenden der Vielzahl von Adsorptionsmitteln befinden,
der Konzentrator für eine chemische Substanz des Weiteren eine Stromzufuhr-Einheit (22) umfasst, die der ersten Elektrode (12, 12a-12i) und der zweiten Elektrode (13a, 13a-13i), die in dem jeweiligen der Vielzahl von Adsorptionsmitteln angeordnet sind, jeweils selektiv einen Strom zuführt.

10. Konzentrator für eine chemische Substanz nach Anspruch 1, der des Weiteren umfasst:
eine Messungs-Einheit, die einen Wert eines durch das Adsorptionsmittel (14, 14a-14i) fließenden Stroms misst; sowie
eine Einheit für Schätzung einer Adsorptionsmenge, die eine Menge der an dem Adsorptionsmittel (14, 14a-14i) adsorbierten chemischen Substanz schätzt, indem sie auf Basis des Stromwertes eine Änderung eines elektrischen Widerstandes des Adsorptionsmittels (14, 14a-14i) bestimmt.

11. Vorrichtung für Erfassung einer chemischen Substanz, die umfasst:
den Konzentrator (20, 20A-20E) für eine chemische Substanz nach Anspruch 1; sowie
eine Erfassungs-Einheit ( 21), die die durch den Konzentrator (20, 20A-20E) für eine chemische Substanz konzentrierte chemische Substanz erfasst.

## Revendications

1. Concentrateur de substance chimique comprenant :
un passage d'écoulement (11) qui permet à un échantillon gazeux contenant une substance chimique de s'écouler à travers le passage d'écoulement ;
une première électrode (12, 12a-12i) disposée sur une première paroi interne (111A) du passage d'écoulement (11) ;
une deuxième électrode (13, 13a-13i) disposée sur la première paroi interne (111A), la deuxième électrode (13, 13a-13i) étant éloignée de la première électrode (12, 12a-12i) ; et
un adsorbant conducteur (14, 14a-14i) en contact avec la première électrode (12, 12a-12i), la deuxième électrode (13, 13a-13i) et la première paroi interne (111A) entre la première électrode (12, 12a-12i) et la deuxième électrode (13, 13a-13i),
dans lequel l'adsorbant (14) est configuré pour adsorber la substance chimique,
l'adsorbant (14) est constitué d'un agrégat de nanofils conducteurs (141),
l'adsorbant (14) comprend des vides (143) pourvus chacun entre les nanofils,
les nanofils adjacents (141) de l'adsorbant (14) sont reliés entre eux par les bases de nanofils adjacents (141) sur la première paroi interne (111A) de manière à conférer à l'adsorbant (14) une conductivité électrique dans son ensemble,
la première électrode et la deuxième électrode sont connectées électriquement via l'adsorbant (14),
l'adsorbant (14, 14a-14i) est configuré pour s'auto-échauffer par effet Joule quand un courant traverse l'adsorbant (14, 14a-14i) via la première électrode (12, 12a-12i) et la deuxième électrode (13, 13a-13i), et
l'adsorbant est configuré pour désorber la substance chimique adsorbée de l'adsorbant (14) en raison de la chaleur générée par l'adsorbant (14).

2. Concentrateur de substance chimique selon la revendication 1, dans lequel les nanofils (141) sont constitués d'oxyde métallique.

3. Concentrateur de substance chimique selon la revendication 1, dans lequel les nanofils (141) sont constitués de nanotubes de carbone revêtus d'oxyde métallique.

4. Concentrateur de substance chimique selon la revendication 1, comprenant en outre une unité de refroidissement (15) pour refroidir l'adsorbant (14, 14a-14i) .

5. Concentrateur de substance chimique selon la revendication 1, dans lequel la première paroi interne (111A) comprend une couche d'isolation thermique (16) en contact avec l'adsorbant (14, 14a-14i).

6. Concentrateur de substance chimique selon la revendication 1, dans lequel le passage d'écoulement (11) comporte une pluralité de parois internes (111A-111C), et la première paroi interne (111A) est une paroi de la pluralité de parois internes (111A-111C).

7. Concentrateur de substance chimique selon la revendication 1, dans lequel l'adsorbant (14, 14a-14i) comprend une pluralité d'adsorbants disposés séparément les uns des autres dans le passage d'écoulement (11).

8. Concentrateur de substance chimique selon la revendication 7, dans lequel la pluralité d'adsorbants est constituée de matériaux différents les uns des autres, ou ont des surfaces modifiées différemment les unes des autres.

9. Concentrateur de substance chimique selon la revendication 7 ou 8,
dans lequel chaque électrode parmi la première électrode (12, 12a-12i) et la deuxième électrode (13, 13a-13i) se trouve sur un adsorbant respectif de la pluralité d'adsorbants,
le concentrateur de substance chimique comprenant en outre une unité d'alimentation de courant (22) qui alimente sélectivement un courant à chaque électrode parmi la première électrode (12, 12a-12i) et la deuxième électrode (13a, 13a-13i) disposées dans l'adsorbant respectif de la pluralité d'adsorbants.

10. Concentrateur de substance chimique selon la revendication 1, comprenant en outre :
une unité de mesure qui mesure la valeur d'un courant circulant à travers l'adsorbant (14, 14a-14i) ; et
une unité d'estimation de la quantité d'adsorption qui estime la quantité de substance chimique adsorbée sur l'adsorbant (14, 14a-14i) en déterminant, sur la base de la valeur du courant, un changement de la résistance électrique de l'adsorbant (14, 14a-14i).

11. Dispositif de détection de substance chimique comprenant :
le concentrateur de substance chimique (20, 20A-20E) selon la revendication 1 ; et
une unité de détection (21) qui détecte la substance chimique concentrée par le concentrateur de substance chimique (20, 20A-20E).
